# EUROPÄISCHE PATENTSCHRIFT

(11) **EP 1 562 596 B1**
(45) Veröffentlichungstag und Bekanntmachung des Hinweises auf die Patenterteilung: **30.08.2006**
(21) Anmeldenummer: 03811744.6
(22) Anmeldetag: 30.10.2003
(51) Int. Cl.: A61K 31/4439, A61K 31/4709, C07D 401/12

(54) **PYRIDINALYKL-AMINOALKYL-1H-INDOL DERIVATE MIT HEMMENDER WIRKUNG AUF DIE 5-HT UND SEROTONIN WIEDERAUFNAHME ALS ANTIDEPRESSIVA UND ANXIOLYTIKA**
DERIVATIVES OF PYRIDINALKYL-AMONOALKYL-1H-INDOLE INHIBITING RECEPTORS 5-HT AND RECAPTURE OF SEROTONIN WHICH CAN BE USED AS ANTIDEPRESSANT AND ANXIOLITIC
DERIVES DE PYRIDINALKYL-AMINOALKYL-1H-INDOLE INHIBANT LES RECEPTEURS 5-HT ET LA RECAPTURE DE LA SEROTONINE, POUVANT SERVIR D'ANTIDEPRESSEURS ET D'ANXIOLYTIQUES

(30) Priorität: 22.11.2002 DE 10254596
(43) Veröffentlichungstag der Anmeldung: 17.08.2005
(73) Patentinhaber: Merck Patent GmbH, 64293 Darmstadt (DE)
(72) Erfinder: HÖLZEMANN, Günter, 64342 Seeheim-Jugenheim (DE); SCHIEMANN, Kai, 64297 Darmstadt (DE); HEINRICH, Timo, 64823 Gross-Umstadt (DE); BÖTTCHER, Henning, 64287 Darmstadt (DE); LEIBROCK, Joachim, 64319 Pfungstadt (DE); VAN AMSTERDAM, Christoph, 64295 Darmstadt (DE); BARTOSZYK, Gerd, 64331 Weiterstadt (DE); SEYFRIED, Christoph, 64342 Seeheim-Jugenheim (DE)
(86) Internationale Anmeldenummer: PCT/EP2003/012081
(87) Internationale Veröffentlichungsnummer: WO 2004/047840

(56) Entgegenhaltungen:
- WO-A-01/98293
- WO-A-96/20191
- WO-A-97/13512
- VACHER, BERNARD ET AL: "Design and Synthesis of a Series of 6-Substituted 2-Pyridinylmethylamine Derivatives as Novel, High-Affinity, Selective Agonists at 5 - HT1A Receptors" JOURNAL OF MEDICINAL CHEMISTRY (1998), 41(25), 5070-5083 , XP002931552
- YAJIMA, TATSUO ET AL: "Conformational preference of the side chain aromatic ring in Cu(II) and Pd(II) complexes of 2N1O-donor ligands" INORGANICA CHIMICA ACTA (2002), 337, 193-202 , XP001179958

## Beschreibung

Die Erfindung betrifft Indolderivate der Formel I 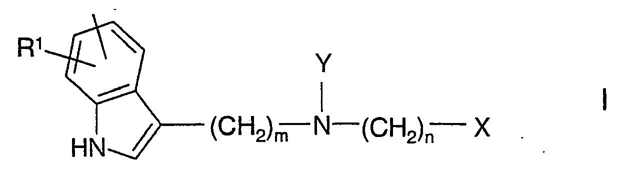 worin
- X: 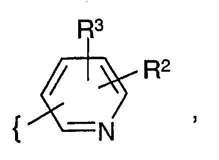
- Y: H oder A',
- R¹: CN oder F
- R²: H, A, Hal, OH, OA, SA, COOH, COOA', CHO, COA', SO₂A', NH₂, NHA, NA₂, CH₂NA₂, NHCOA, NHCOAr, NHCOOA, NHSO₂A, NHSO₂Ar, CH₂NHSO₂A, NHCONH₂, NHCONHA, NHCONA₂, NHCONHAr, CONH₂, CONHA, CONA₂, SO₂NH₂, SO₂NHA, SO₂NA₂, CH₂SO₂NH₂, CH₂SO₂NHA, CH₂SO₂NA₂, [C(R⁴R^{4'})]ₚCN, [C(R⁴R^{4'})]ₚCF₃, [C(R⁴R^{4'})]ₚCOR⁴, [C(R⁴R⁴)]ₚAr, -O-[C(R⁴R^{4'})]ₚAr oder [C(R⁴R^{4'})]ₚHet,
- R³: H, A, [C(R⁴R^{4'})]ₚAr oder [C(R⁴R^{4'})]ₚHet,
- R² und R³: zusammen auch -CH=CH-CH=CH-, -CH=CH-CH₂-CH₂- oder -CH₂-CH₂-CH=CH- worin 1 oder 2 CH- und/oder CH₂-Einheiten durch N ersetzt sein können und/oder 1, 2, 3 oder 4 H-Atome durch Hal, A, OH, OA, NH₂, NO₂, CN, COOH, COOA, CONH₂, NHCOA, NHCONH₂, NHSO₂A, CHO, COA, SO₂NH₂ oder S(O)ₒA substituiert sein können,
-O-CH₂-O- oder -O-CH₂-CH₂-O-,
- R⁴, R^{4'}: jeweils unabhängig voneinander H, A, OH, OA, NH₂, NHA, NA₂ oder NHCOOA',
- Ar: unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂ oder S(O)ₒA, substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =NH, Hal, A, -(CH₂)ₒ-Ar, -(CH₂)ₒ-Cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ und/oder S(O)ₒA substituiert sein kann,
- A: unverzweigtes oder verzweigtes Alkyl mit 1-10 C-Atomen, worin eine oder zwei CH₂-Gruppen durch -CH=CH-Gruppen und/oder auch 1-7 H-Atome durch F und/oder Cl ersetzt sein können,
- A': Alkyl mit 1 bis 6 C-Atomen oder Benzyl,
- Hal: F, Cl, Br oder 1 und
- m: 2, 3, 4, 5 oder 6
- n: 1, 2, 3 oder 4,
- o: 0, 1 oder 2,
- p: 0, 1, 2, 3, 4, 5 oder 6,
bedeuten,
sowie ihre pharmazeutisch verwendbaren Salze, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden.

Heterocyclische Aminoalkylpyridinderivate sind in der WO 01/98293 beschrieben. Die vorliegende Erfindung ist hierzu als Auswahlerfindung zu betrachten.
Andere Indolderivate kennt man aus WO 94/24127, WO 90/05721 oder aus JP 05043544.

Es wurde gefunden, dass die erfindungsgemäßen Verbindungen der Formel I und ihre physiologisch unbedenklichen Säureadditionssalze bei guter Verträglichkeit wertvolle pharmakologische Eigenschaften besitzen, da sie Wirkungen auf das Zentralnervensystem, insbesondere 5-HTreuptake hemmende Wirkungen, aufweisen, indem sie die serotoninerge Transmission beeinflussen. Insbesondere weisen sie eine starke Affinität zu den 5-HT_{1A}- und teilweise zu den 5-HT_{1D}- Rezeptoren auf.

Da die Verbindungen auch die Serotonin-Wiederaufnahme hemmen, eignen sie sich insbesondere als Antidepressiva und Anxiolytika. Die Verbindungen zeigen serotonin-agonistische und -antagonistische Eigenschaften. Sie hemmen die Bindung von tritiierten Serotoninliganden an hippocampale Rezeptoren (Cossery et al., European J. Pharmacol. 140 (1987), 143-155) und hemmen die synaptosomale Serotoninwiederaufnahme (Sherman et al., Life Sci. 23 (1978), 1863-1870).
Zum ex-vivo Nachweis der Serotoninwiederaufnahmehemmung wird die synaptosomale Aufnahmehemmung (Wong et al., Neuropsychopharmacol. 8 (1993), 23-33) und der p-Chloramphetaminantagonismus (Fuller et al., J. Pharmacol. Exp.Ther. 212 (1980), 115-119) herangezogen. Die 5-HT_{1D-}Affinität kann beispielweise bestimmt werden nach der von Pauwels und Palmier in Neuropharmacology, 33, 67 (1994) beschriebenen Methode.

Die Bindungseigenschaften der Verbindungen der Formel I lassen sich durch bekannte 5-HT_{1A}-(Serotonin)-Bindungstests bestimmen (5-HT_{1A-}(Serotonin)-Bindungstest: Matzen et al., J. Med. Chem., 43, 1149-1157, (2000) insbesondere Seite 1156 mit Verweis auf Eur. J. Pharmacol.: 140, 143-155 (1987).

Die erfindungsgemäßen Verbindungen können zur Behandlung von Krankheiten eingesetzt werden, die mit dem Serotonin- Neurotransmittersystem verbunden sind und bei denen hochaffine Serotoninrezeptoren (5-HT_{1A}-Rezeptoren) und/oder 5-HT_{1D}-Rezeptoren beteiligt sind.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler lschämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD), Angstzuständen, Panikattacken, Psychosen, Anorexie, wahnhaften Zwangsvorstellungen, Migräne, der Alzheimer Krankheit, Schlafstörungen, tardiver Dyskinesien, Lernstörungen, altersabhängiger Erinnerungsstörungen, Essstörungen wie Bulimie, Drogenmissbrauch und/oder Sexualfunktionsstörungen.

Eine wichtige Indikation für die Verabreichung der Verbindung der allgemeinen Formel I sind Psychosen jeder Art, insbesondere auch Geisteskrankheiten aus dem Formenkreis der Schizophrenie. Darüber hinaus können die Verbindungen auch zur Verminderung von kognitiven Leistungsstörungen, also zur Verbesserung der Lernfähigkeit und des Gedächtnisses, eingesetzt werden. Auch zur Bekämpfung der Symptome von Morbus-Alzheimer sind die Verbindungen der allgemeinen Formel I geeignet. Darüber hinaus eignen sich die erfindungsgemäßen Substanzen der allgemeinen Formel I zur Prophylaxe und Kontrolle von Hirninfarkten (Apoplexia cerebri), wie Hirnschlag und zerebrale Ischemie. Ferner kommen die Substanzen zur Behandlung von Krankheiten wie krankhafte Angstzustände, Übererregung, Hyperaktivität und Ausmerksamkeitsstörungen bei Kindern und Jugendlichen, tiefgreifende Entwicklungsstörungen und Störungen des Sozialverhaltens mit geistiger Retardierung, Depression, Zwangserkrankungen im engeren (OCD) und weiteren Sinne (OCSD) bestimmte sexuelle Funktionsstörungen, Schlafstörungen und Störungen in der Nahrungsaufnahme, sowie solcher Psychiatrischer Symptome in Rahmen der Altersdemenz und der Demenz vom Alzheimer-Typ, also Kranheiten des zentralen Nervensystems im weitesten Sinn, in Frage.

Die Verbindungen der Formel I eignen sich ebenfalls zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden.

Extrapyramidale Bewegungserkrankungen sind beispielsweise idiopathische Parkinson'sche Krankheit, Parkinson-Syndrom, dyskinetische choreatische oder dystonische Syndrome, Tremor, Gilles de la Torette Syndrom, Ballismus, Muskelkrampf, Restless Legs Syndrom, Wilsons-Krankheit, Lewy bodies Dementia, Huntington und Tourette Syndrom.

Die erfindungsgemäßen Verbindungen eignen sich insbesondere auch zur Behandlung von neurodegenerativen Erkrankungen, wie z.B. Lathyrismus, Alzheimer, Parkinson, und Huntington.

Die Verbindungen der Formel I eignen sich insbesondere zur Behandlung von Nebenwirkungen, die bei der Behandlung der idiopathischen Parkinson-Krankheit mit konventionellen Parkinson-Medikamenten auftreten. Sie können deshalb auch als Zusatztherapie (add-on therapy) bei der Behandlung der Parkinson-Krankheit verwendet werden. Bekannte Parkinson-Medikamente sind Arzneimittel wie L-Dopa (Levodopa) und L-Dopa kombiniert mit Benserazid oder Carbidopa, Dopamin Agonisten wie Bromocriptin, Apomorphin, Cabergolin, Pramipexol, Ropinirol, Pergolid, Dihydro-α-ergocriptin oder Lisurid und alle Medikamente, die eine Stimulierung des Dopamin-Rezeptors bewirken, Inhibitoren der Catechol-O-methyl-Transferase (COMT) wie Entacapon oder Tolcapon, Inhibitoren der Monoamin Oxidase (MAO) wie Selegilin und Antagonisten von N-Methyl-D-aspartat (NMDA) Rezeptoren wie Amantadin oder Budipin.

Die Verbindungen der allgemeinen Formel I und ihre verträglichen Salze und Solvate können somit als aktive Inhaltsstoffe für Arzneimittel wie Anxiolytika, Antidepressiva, Neuroleptika und/oder Antihypertensiva eingesetzt werden.

Ein Maß für die Aufnahme eines Arzneimittelwirkstoffs in einen Organismus ist seine Bioverfügbarkeit.
Wird der Arzneimittelwirkstoff in Form einer Injektionslösung dem Organismus intravenös zugefügt, so liegt seine absolute Bioverfügbarkeit, d.h. der Anteil des Pharmakons, der unverändert im systemischen Blut, d.h. in den großen Kreislauf gelangt, bei 100%.
Bei oraler Vergabe eines therapeutischen Wirkstoffs liegt der Wirkstoff in der Regel als Feststoff in der Formulierung vor und muß sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al, J. Pharm. Sciences, 1999, 88, 313-318 erhalten werden.
Ein weiteres Maß für die Resorbierbarkeit eines therapeutischen Wirkstoffes ist der logD-Wert, denn dieser Wert ist ein Maß für die Lipophilie eines Moleküls.

Soweit die Verbindungen der allgemeinen Formel I optisch aktiv sind, umfaßt die Formel I sowohl jede isolierte optische Antipode als auch die entsprechenden gegebenenfalls racemischen Gemische in jeder denkbaren Zusammensetzung.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung sind die Verbindungen der Formel I und ihre Salze und Solvate nach Anspruch 1 sowie ein Verfahren zur Herstellung von Verbindungen der Formel I sowie ihrer Salze und Solvate, dadurch gekennzeichnet, daß man
a) eine Verbindung der Formel II 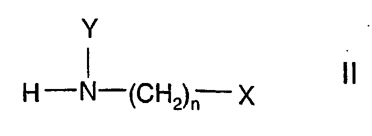 worin
   X, Y und n die in Anspruch 1 angegebenen Bedeutungen haben,
   mit einer Verbindung der Formel (II 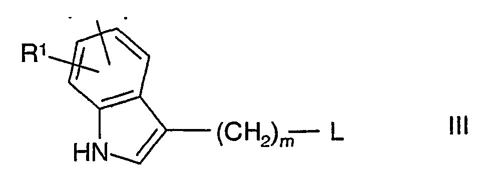 worin
   - L: Cl, Br oder I bedeutet und R¹ und m die in Anspruch 1 angegebenen Bedeutungen haben,
   umsetzt,
   oder
b) eine Verbindung der Formel IV

   X-(CH₂)ₙ-L IV

   worin
   - L: Cl, Br oder I bedeutet und X und n die in Anspruch 1 angegebenen Bedeutungen haben,
   mit einer Verbindung der Formel V 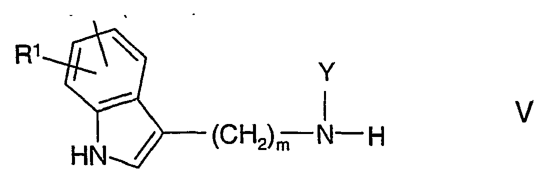 worin
   R¹, Y und m die in Anspruch 1 angegebenen Bedeutungen haben,
   umsetzt,
   und/oder
   eine basische oder saure Verbindung der Formel I durch Behandeln mit einer Säure oder Base in eines ihrer Salze oder Solvate umwandelt.

Gegenstand der Erfindung sind auch Mischungen der erfindungsgemäßen Verbindungen der Formel 1, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
A bedeutet auch Cycloalkyl.
Cycloalkyl bedeutet vorzugsweise Cyclopropyl, Cyclobutyl, Cylopentyl, Cyclohexyl oder Cycloheptyl.

A' bedeutet bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl oder Benzyl.

-COA bzw. -COA'(Acyl) bedeutet vorzugsweise Acetyl, Propionyl, ferner auch Butyryl, Pentanoyl, Hexanoyl oder z.B. Benzoyl.
Hal bedeutet vorzugsweise F, Cl oder Br, aber auch 1.

OA bedeutet vorzugsweise z.B. Methoxy, Ethoxy, Propoxy, Isopropoxy, Butoxy oder tert.-Butoxy.

R¹ bedeutet CN oder F, ganz besonders bevorzugt CN.
m bedeutet vorzugsweise 4.
n bedeutet vorzugsweise 1 oder 2.

R² bedeutet vorzugsweise H, Hal, A, SA, CN, CONH₂, COOA', -O-[C(R⁴R^{4'})]ₚAr, [C(R⁴R^{4'})]ₚAr oder [C(R⁴R^{4'})]ₚHet, wobei Het vorzugsweise unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl Imidazolyl, Thiazolyl, Triazolyl, Tetrazolyl, Chinolyl oder Isochinolyl bedeutet und Ar vorzugsweise Phenyl bedeutet.

R³ bedeutet vorzugsweise H, A, Hal oder CN.
R² und R³ bedeuten vorzugsweise zusammen auch -CH=CH-CH=CH-.

R⁴ und R^{4'} bedeuten vorzugsweise H.

Ar bedeutet z.B. unsubstituiertes Phenyl, Naphthyl oder Biphenyl, weiterhin vorzugsweise z.B. durch A, Fluor, Chlor, Brom, lod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.
Ar bedeutet ganz besonders bevorzugt Phenyl.
Het bedeutet, von den möglichen Substituenten abgesehen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-,4-,5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-lsochinolyl, 3-,4-,5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Chromenyl.
Die heterocyclischen Reste können auch teilweise oder vollständig hydriert sein.

Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet besonders bevorzugt unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl Imidazolyl, Thiazolyl, Triazolyl, Tetrazolyl, Chinolyl oder Isochinolyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis lh ausgedrückt werden, die der Formel I entsprechen und worin die nicht näher bezeichneten Reste die bei der Formel I angegebene Bedeutung haben, worin jedoch
- in Ia: R¹ CN oder F, bedeuten;
- in Ib: R⁴, R^{4'} H bedeuten;
- in Ic: R² H, Hal, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-, bedeuten;
- in Id: Het unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl, Imidazolyl, Thiazolyl, Triazolyl, Tetrazolyl, Chinolyl oder Isochinolyl bedeutet;
- in Ie: Ar unsubstituiertes oder ein-, zwei- oder dreifach durch Hal substituiertes Phenyl bedeutet;
- in If: X 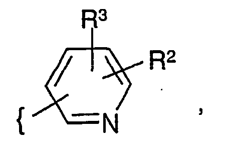
R¹ CN oder F,
R² H, Hal, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-,
Het unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl, Imidazolyl, Thiazolyl, Triazolyl, Tetrazolyl, Chinolyl oder Isochinolyl,
Ar unsubstituiertes oder ein-, zwei- oder dreifach durch Hal substituiertes Phenyl,
p 0 oder 1, bedeuten;
- in Ig: X 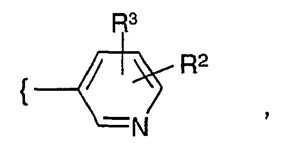
R¹ CN oder F,
R² H, Hal, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-,
Ar Phenyl,
p 0 oder 1, bedeuten;
- in Ih: X 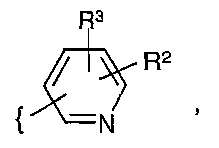
R¹ CN oder F,
R² H, Hal, A, SA, CN, CONH₂, COOA', -O-[C(R⁴R^{4'})]ₚAr, [C(R⁴R^{4'})]ₚAr,
R³ H, A, Hal oder CN,
R² und R³ zusammen auch -CH=CH-CH=CH-,
R⁴, R^{4'} H,
Ar Phenyl,
p 0 oder 1, bedeuten;
sowie ihre pharmazeutisch verwendbaren Salze, Solvate und Stereoisomere, einschließlich deren Mischungen in allen Verhältnissen.

Die Verbindungen der Formel I nach Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I nach Anspruch 1 umsetzt.

Die Ausgangsverbindungen der Formel II und III sind in der Regel bekannt. Sind sie neu, so können sie aber nach an sich bekannten Methoden hergestellt werden.

Verbindungen der Formel I können durch nucleophile Substitution der Abgangsgruppe L der Verbindungen der Formel III, wobei L Cl, Br oder I bedeutet, durch den Amin-Stickstoff der Verbindung der Formel II unter Standardbedingungen hergestellt werden.

Die Reaktionsbedingungen für nucleophile Substitutionen, wie zuvor beschrieben, sind dem Fachmann hinreichend bekannt, siehe auch Organikum, 17. Auflage, Deutscher Verlag für Wissenschaften, Berlin 1988.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Ethyldiisopropylamin, Triethylamin, Dimethylanilin, Pyridin oder Chinolin oder eines Überschusses der Komponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können ferner erhalten werden, indem man Verbindungen der Formel IV mit Verbindungen der Formel V umsetzt. Die Ausgangsverbindungen der Formel IV und V sind in der Regel bekannt. Sind sie neu, so können sie aber nach an sich bekannten Methoden hergestellt werden.
Die Reaktionsbedingungen sind analog denen der Umsetzung zwischen Verbindungen der Formel II und Verbindungen der Formel III.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden.
Andererseits können Verbindungen der Formel I mit Basen (z.B. Natrium- oder Kaliumhydroxid oder -carbonat) in die entsprechenden Metall-, insbesondere Alkalimetall- oder Erdalkalimetall- oder in die entsprechenden Ammoniumsalze umgewandelt werden.

Gegenstand der Erfindung sind auch die Verbindungen der Formel I nach Anspruch 1 und ihre physiologisch unbedenklichen Salze oder Solvate als Arzneimittelwirkstoffe.

Weiterhin sind Gegenstand der Erfindung Verbindungen der Formel I und ihre physiologisch unbedenklichen Salze oder Solvate als 5HT_{1A}- und/oder 5HT_{1D}- Agonisten und als Inhibitoren der 5-HT-Wiederaufnahme.

Gegenstand der Erfindung sind auch die Verbindungen der Formel I nach Anspruch 1 und ihre physiologisch unbedenklichen Salze oder Solvate zur Anwendung bei der Bekämpfung von Krankheiten.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Ein weiterer Gegenstand der Erfindung ist die Verwendung einer Verbindung der allgemeinen Formel I und/oder ihre pharmazeutisch verwendbaren Salze, Solvate und Stereoisomere, einschließlich deren Mischungen in allen Verhältnissen zur Herstellung eines Arzneimittels, welches sich zur Behandlung von menschlichen oder tierischen Krankheiten, insbesondere von Krankheiten des zentralen Nervensystems wie krankhafte Spannungszustände, Depression und/oder Psychosen, zur Verminderung von Nebenwirkungen bei der Behandlung von Bluthochdruck (z.B. mit a-Methyldopa), zur Behandlung von endoklinologischen und/oder gynäkologischen Krankheiten, z.B. zur Behandlung von Agromegalie, Hypogonadismus, sekundärer Amenorrhoe, dem postmenstruellen Syndrom und unerwünschter Laktation in der Pubertät und zur Prophylaxe und Therapie von zerebralen Krankheiten (z.B. von Migräne) insbesondere in der Gereatrie in ähnlicher Weise wie bestimmte Ergot-Alkaloide und zur Kontrolle und Prophylaxe von Hirninfarkt (Apoplexia cerebri) wie Hirnschlag und zerebraler Ischaemie, zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden, eignet. Darüber hinaus sind die pharmazeutischen Zubereitungen und Arzneimittel, die eine Verbindung der allgemeinen Formel I enthalten, zur Verbesserung des kognitiven Leistungsvermögens und zur Behandlung von Morbus-Alzheimer-Symptomen geeignet.

Insbesondere eigngen sich solche Arzneimittel zur Behandlung von Geisteskrankheiten aus dem Formenkreis der Schizophrenie und zur Bekämpfung von psychotischen Angstzuständen. Der Begriff Behandlung schließt im Rahmen der Erfindung Prophylaxe und Therapie menschlicher oder tierischer Krakheiten ein.
Gegenstand der Erfindung ist weiterhin die Verwendung von Verbindungen der Formel I und/oder ihrer pharmazeutisch verwendbaren Salze, Solvate und Stereoisomere, einschließlich deren Mischungen in allen Verhältnissen, zur Herstellung eines Arzneimittels zur Bekämpfung von Krankheiten, die mit dem Serotonin-Neurotransmittersystem verbunden sind und bei denen hochaffine Serotoninrezeptoren (5-HT_{1A}-Rezeptoren) und/oder 5-HT_{1D} -Rezeptoren beteiligt sind.

Gegenstand der Erfindung ist weiterhin die Verwendung von Verbindungen der Formel I und/oder ihrer pharmazeutisch verwendbaren Salze, Solvate und Stereoisomere, einschließlich deren Mischungen in allen Verhältnissen, zur Herstellung eines Arzneimittels als Anxiolytikum, Antidepressivum, Neuroleptikum und/oder Antihypertensivum.

Die Substanzen der allgemeinen Formel I werden normalerweise analog zu bekannten, kommerziell erhältlichen pharmazeutischen Zubereitungen (z.B. von Bromocriptin und Dihydroergocornin), vorzugsweise in Dosen von zwischen 0,2 und 500 mg, insbesondere von zwischen 0,2 und 15 mg pro Dosiseinheit verabreicht. Die tägliche Dosiseinheit ist zwischen 0,001 und 10 mg pro kg Körpergewicht. Geringe Dosen (von zwischen 0,2 und 1 mg pro Dosiseinheit, 0,001 bis 0,005 mg pro kg Körpergewicht) sind besonders geeignet für pharmazeutische Zubereitungen zur Behandlung von Migräne. Eine Dosis zwischen 10 und 50 mg pro Dosiseinheit wird für andere Indikationen bevorzugt. Allerdings hängt die zu verabreichende Dosis von einer Vielzahl von Faktoren ab, z.B. von der Wirksamkeit der entsprechenden Komponente, dem Alter, dem Körpergewicht und der allgemeinen Verfassung des Patienten.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt duch Chromatographie an Kieselgel, durch präparative HPLC und/oder durch Kristallisation. Die gereinigten Verbindungen werden gegebenenfalls gefriergetrocknet.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ (wenn nichts anderes angegeben) |

### Beispiel 1

Die Synthese des Indolbausteins erfolgt analog nachstehendem Schema:

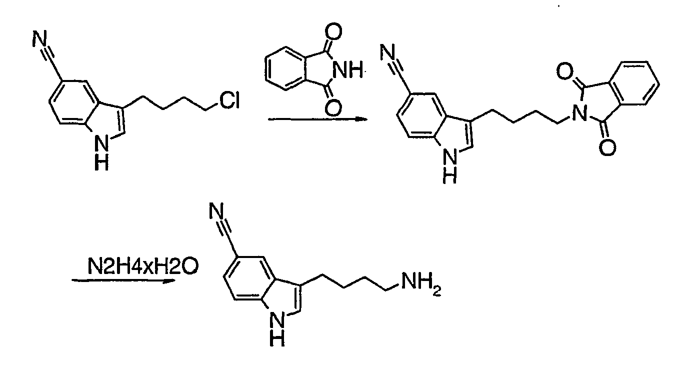

Die Synthese von 3-{4-[(5-Brom-pyridin-3-ylmethyl)-amino]-butyl}-1*H*-indol-5-carbonitril erfolgt analog nachstehendem Schema: 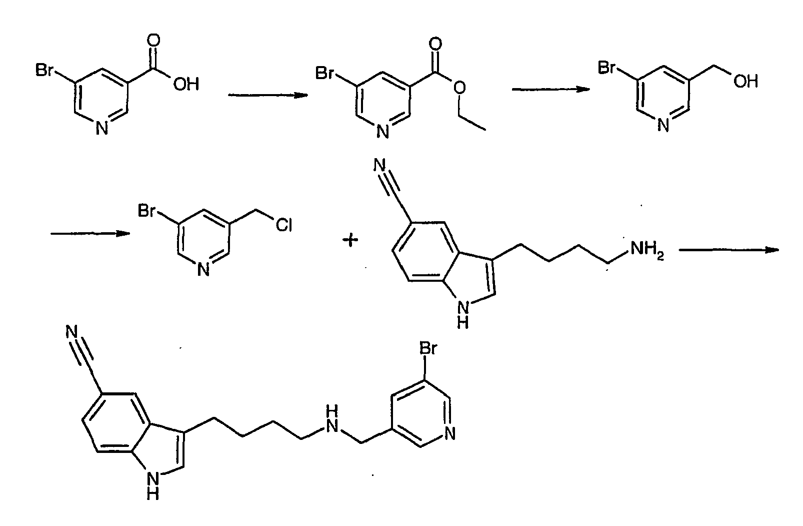
1.1 1,3 g 5-Brom-nicotinsäureethylester werden in 40 ml tert.-Butanol vorgelegt und 427 mg Natriumborhydrid zugegeben. Das Ganze wird eine Stunde unter Rückfluß (100° C) und unter N₂ erhitzt. Dann werden 6 ml Methanol zugegeben. Das Gemisch wird über Nacht gerührt. Zur Reaktionslösung wird 10 ml Wasser hinzugegeben. Danach wird mit Dichlomethan extrahiert, mit Natriumsulfat getrocknet und einrotiert. Man erhält 300 mg (5-Brom-pyridin-3-yl)-methanol.
1.2 300 mg des zuvor gewonnen Rohproduktes (5-Brom-pyridin-3-yl)-methanol werden in 20 Toluol gelöst. Es werden 0,2 ml Thionylchlorid zugegeben und eine Stunde bei 100° C gerührt. Danach wird einrotiert. Der ölige Rückstand enthält 3-Brom-5-chlormethyl-pyridin (315 mg).
1.3 300 mg 3-Brom-5-chlormethyl-pyridin und 331 mg 3-(4-Aminobutyl)-1*H*-indol-5-carbonitril werden in Acetonitril zusammengegeben.

Danach werden 415 mg Kaliumcarbonat und eine Spatelspitze Kaliumjodid zugegeben. Es wird über Nacht unter Rückfluß gekocht. Das Gemisch wird einrotiert und mit Wasser versetzt. Es wird zweimal mit Essigester extrahiert, mit Natriumsulfat getrocknet, filtriert und einrotiert.
390 mg des Rohproduktes werden mit Hilfe der präparativen HPLC gereinigt:

| | |
|---|---|
| HPLC-Säule: | RP 18 (15 mm) Lichrosorb |
| Fließmittel: | A 98 H₂O, 2 CH₃CN, 0,1 % TFA |
| | B 10 H₂O, 90 CH₃CN, 0,1% TFA |
| UV-Detektion: | 225 NM; 1 range |
| Fluß: | 10 ml |

Fraktion 19-23 enthält die gewünschte Verbindung 3-{4-[(5-Brom-pyridin-3-ylmethyl)-amino]-butyl}-1*H*-indol-5-carbonitril ; bis-TFA-Salz.
Ausbeute: 143 mg
HPLC-ESI-MS (M+H)⁺ 383.

### Beispiel 2

### Herstellung von 3-{4-[(2-Chlor-6-methyl-pyridin-4-ylmethyl)-amino]-butyl}-1H-indol-5-carbonitril:

2.1 1,94 g 2-Chlor-6-methyl-isonicotinsäureethylester werden in 36 ml tert.-Butanol vorgelegt, 0,93 g Natriumborhydrid zugegeben und unter Stickstoff zum Rückfluss erhitzt (Badtemperatur ca. 120C). Nach 1h werden 9 ml Methanol langsam zugegeben, über Nacht am Rückfluss gekocht. Man arbeitet wie üblich auf und erhält 1,15 g (2-Chlor-6-methyl-pyridin-4-yl)-methanol.
2.2 1,14 g (2-Chlor-6-methyl-pyridin-4-yl)-methanol werden in 50 ml Toluol vorgelegt und unter Eiskühlung werden langsam 1,05 ml Thionylchlorid zugetropft. Es wird über Nacht unter Rückfluss gekocht. Nach Abkühlen wird das Lösungsmittel entfernt. Man erhält 2-Chlor-4-chlormethyl-6-methyl-pyridin als braunes Öl (1,1 g).
2.3 213 mg 2-Chlor-4-chlormethyl-6-methyl-pyridin und 250 mg 3-(4-Amino-butyl)-1*H*-indol-5-carbonitril werden in Acetonitril zusammengegeben. Dazu werden 276 mg Kaliumcarbonat und ca. 3 mg Kaliumjodid gegeben. Das Gemisch wird über Nacht am Rückfluss gekocht. Es wird abgekühlt und danach das Lösungsmittel entfernt. Danach wird mit 20ml Wasser und 20ml Ethylacetat versetzt.
   Man arbeitet wie üblich auf und erhält einen braunen Rückstand (0,4 g). Die Reinigung erfolgt durch Flash-Chromatographie (Fließmittel:
   CH₂Cl₂/MeOH 97:3). Man erhält 150 mg 3-{4-[(2-Chlor-6-methyl-pyridin-4-ylmethyl)-amino]-butyl}-1*H*-indol-5-carbonitril, HPLC-ESI-MS (M+H)⁺ 353.

### Beispiel 3

### Herstellung von 3-{4-[(Chinolin-3-ylmethyl)-amino]-butyl}-1H-indol-5-carbonitril:

126 mg Chinolin-3-carboxaldehyd und 200 mg 3-(4-Amino-butyl)-1*H*-indol-5-carbonitril werden in einer Mischung von 5 ml 1,2-Dichlorethan und 2,5 ml THF zusammengegeben. Es werden 55 mg Eisessig zugesetzt und 3 Stunden bei Raumtemperatur gerührt. Nun gibt man 380 mg NaB(OAc)₃ zu und rührt 2 Tage bei RT weiter.
Der Ansatz wird mit gesättigter NaHCO₃-Lösung versetzt, 2mal mit Essigester extrahiert, über Na₂SO₄ getrocknet, filtriert und einrotiert. Chromatographie am Flash-Master mit EE/MeOH als Eluent ergeben 73 mg des gewünschten Produktes, HPLC-MS (M+H)⁺ 355.

Analog erhält man die nachstehenden Verbindungen
3-{4-[(Chinolin-2-ylmethyl)-amino]-butyl}-1*H*-indol-5-carbonitril, HPLC-MS (M+H)⁺ 355;
3-{4-[(Chinolin-4-ylmethyl)-amino]-butyl}-1*H*-indol-5-carbonitril, HPLC-MS (M+H)⁺ 355.

### Beispiel 4

### Analog Beispiel 1 erhält man die nachstehenden Verbindungen

3-{4-[(Pyridin-3-ylmethyl)-amino]-butyl}-1*H*-indol-5-carbonitril, Dihydrochlorid, HPLC-MS (M+H)⁺ 305;
3-{4-[(Pyridin-2-ylmethyl)-amino]-butyl}-1*H*-indol-5-carbonitril, Dihydrochlorid, HPLC-MS (M+H)⁺ 305;
3-{4-[(Pyridin-4-ylmethyl)-amino]-butyl}-1*H* indol-5-carbonitril, Dihydrochlorid, HPLC-MS (M+H)⁺ 305;
3-(4-{[5-(4-Fluorphenyl)-pyridin-3-ylmethyl]-amino}-butyl)-1*H*-indol-5-carbonitril, HPLC-MS (M+H)⁺ 399;
3-[4-(2-Pyridin-4-yl-ethylamino]-butyl]-1*H*-indol-5-carbonitril, HPLC-MS (M+H)⁺ 319;
3-{4-[(2,6-Dichlor-pyridin-4-ylmethyl)-amino]-butyl}-1*H*-indol-5-carbonitril, HPLC-MS (M+H)⁺ 373;
3-{4-[(2-Chlor-pyridin-4-ylmethyl)-amino]-butyl}-1*H*-indol-5-carbonitril, HPLC-MS (M+H)⁺ 339;
3-{4-[(2-Methyl-pyridin-3-ylmethyl)-amino]-butyl}-1*H*-indol-5-carbonitril, HPLC-MS (M+H)⁺ 318;
3-{4-[(6-Chlor-pyridin-3-ylmethyl)-amino]-butyl}-1*H*-indol-5-carbonitril, bis-TFA-Salz, HPLC-MS (M+H)⁺ 339;
3-(4-{[2-(4-Chlor-phenoxy)-pyridin-3-ylmethyl]-amino}-butyl)-1*H*-indol-5-carbonitril, TFA-Salz, HPLC-MS (M+H)⁺ 431;
3-{4-[(2-Methylsulfanyl-pyridin-3-ylmethyl)-amino]-butyl}-1*H*-indol-5-carbonitril, TFA-Salz, HPLC-MS (M+H)⁺ 351;
3-{4-[(2,5-Dichlor-pyridin-3-ylmethyl)-amino]-butyl}-1*H*-indol-5-carbonitril, bis-TFA-Salz, HPLC-MS (M+H)⁺ 374;
3-{4-[(2,6-Dichlor-pyridin-3-ylmethyl)-amino]-butyl}-1*H*-indol-5-carbonitril, bis-TFA-Salz, HPLC-MS (M+H)⁺ 374;
3-{4-[(5-Methyl-pyridin-3-ylmethyl)-amino]-butyl}-1*H*-indol-5-carbonitril, bis-TFA-Salz, HPLC-MS (M+H)⁺ 318;
3-{4-[(6-Trifluormethyl-pyridin-3-ylmethyl)-amino]-butyl}-1*H*-indol-5-carbonitril, HPLC-MS (M+H)⁺ 373;
3-{4-[(4-Phenyl-pyridin-2-ylmethyl)-amino]-butyl}-1*H*-indol-5-carbonitril, HPLC-MS (M+H)⁺ 381;
3-{4-[(4-Phenyl-pyridin-3-ylmethyl)-amino]-butyl)-1*H*-indol-5-carbonitril, HPLC-MS (M+H)⁺ 381;
3-{4-[(5-Cyan-6-methylsulfanyl-pyridin-2-ylmethyl)-amino]-butyl}-1*H* indol-5-carbonitril, HPLC-MS (M+H)⁺ 376;
3-{4-[(5-Phenyl-pyridin-3-ylmethyl)-amino]-butyl}-1*H*-indol-5-carbonitril, HPLC-MS (M+H)⁺ 381;
3-{4-[(5-Phenyl-pyridin-2-ylmethyl)-amino]-butyl}-1*H* indol-5-carbonitril, HPLC-MS (M+H)⁺ 381;
3-[4-(Methyl-pyridin-3-ylmethyl-amino)-butyl]-1*H*-indol-5-carbonitril, HPLC-MS (M+H)⁺ 319.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: KapseIn

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel 1 in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer Nacl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 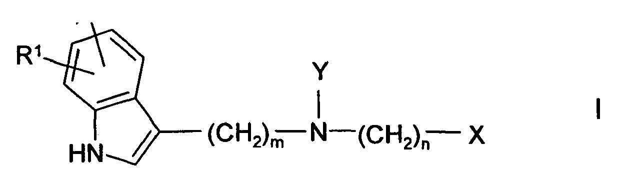 worin
X 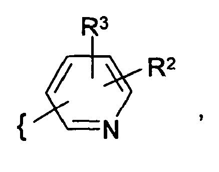
Y H oder A',
R¹ CN oder F,
R² H, A, Hal, OH, OA, SA, COOH, COOA', CHO, COA', SO₂A', NH₂, NHA, NA₂, CH₂NA₂, NHCOA, NHCOAr, NHCOOA, NHSO₂A, NHSO₂Ar, CH₂NHSO₂A, NHCONH₂, NHCONHA, NHCONA₂, NHCONHAr, CONH₂, CONHA, CONA₂, SO₂NH₂, SO₂NHA, SO₂NA₂, CH₂SO₂NH₂, CH₂SO₂NHA, CH₂SO₂NA₂, [C(R⁴R^{4'})]ₚCN, [C(R⁴R^{4'})]ₚCF₃, [C(R⁴R^{4'})]ₚCOR⁴, [C(R⁴R^{4'})]ₚAr, -O-[C(R⁴R^{4'})]ₚAr oder [C(R⁴R^{4'})]ₚHet,
R³ H, A, [C(R⁴R^{4'})]ₚAr oder [C(R⁴R^{4'})]ₚHet,
R² und R³ zusammen auch -CH=CH-CH=CH-, -CH=CH-CH₂-CH₂- oder -CH₂-CH₂-CH=CH- worin 1 oder 2 CH- und/oder CH₂-Einheiten durch N ersetzt sein können und/oder 1, 2, 3 oder 4 H-Atome durch Hal, A, OH, OA, NH₂, NO₂, CN, COOH, COOA, CONH₂, NHCOA, NHCONH₂, NHSO₂A, CHO, COA, SO₂NH₂ oder S(O)ₒA substituiert sein können,
-O-CH₂-O- oder -O-CH₂-CH₂-O-,
R⁴, R^{4'} jeweils unabhängig voneinander H, A, OH, OA, NH₂, NHA, NA₂ oder NHCOOA',
Ar unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂ oder S(O)ₒA, substituiertes Phenyl, Naphthyl oder Biphenyl,
Het einen ein- oder zweikernigen gesättigten, ungesättigten oder aromatischen Heterocyclus mit 1 bis 4 N-, O- und/oder S- Atomen, der unsubstituiert oder ein-, zwei- oder dreifach durch Carbonylsauerstoff, =S, =NH, Hal, A, -(CH₂)ₒ-Ar, -(CH₂)ₒ-Cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ und/oder S(O)ₒA substituiert sein kann,
A unverzweigtes oder verzweigtes Alkyl mit 1-10 C-Atomen, worin eine oder zwei CH₂-Gruppen durch -CH=CH-Gruppen und/oder auch 1-7 H-Atome durch F und/oder Cl ersetzt sein können,
A' Alkyl mit 1 bis 6 C-Atomen oder Benzyl,
Hal F, Cl, Br oder I und
m 2, 3, 4, 5 oder 6
n 1, 2, 3 oder 4,
o 0, 1 oder 2,
p 0, 1, 2, 3, 4, 5 oder 6,
bedeuten,
sowie ihre pharmazeutisch verwendbaren Salze, Solvate und Stereoisomere, einschließlich deren Mischungen in allen Verhältnissen.

2. Verbindungen nach Anspruch 1, worin
R⁴, R^{4'} H bedeuten,
sowie ihre pharmazeutisch verwendbaren Salze, Solvate und Stereoisomere, einschließlich deren Mischungen in allen Verhältnissen.

3. Verbindungen gemäß Anspruch 1 oder 2, worin
R² H, Hal, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-,
bedeuten,
sowie ihre pharmazeutisch verwendbaren Salze, Solvate und Stereoisomere, einschließlich deren Mischungen in allen Verhältnissen.

4. Verbindungen gemäß einem oder.mehreren der Ansprüche 1-3, worin
Het unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl, Imidazolyl, Thiazolyl, Triazolyl, Tetrazolyl, Chinolyl oder Isochinolyl
bedeutet,
sowie ihre pharmazeutisch verwendbaren Salze, Solvate und Stereoisomere, einschließlich deren Mischungen in allen Verhältnissen.

5. Verbindungen gemäß einem oder mehreren der Ansprüche 1-4, worin
Ar unsubstituiertes oder ein-, zwei- oder dreifach durch Hal substituiertes Phenyl
bedeutet,
sowie ihre pharmazeutisch verwendbaren Salze, Solvate und Stereoisomere, einschließlich deren Mischungen in allen Verhältnissen.

6. Verbindungen gemäß einem oder mehreren der Ansprüche 1-5, worin
X 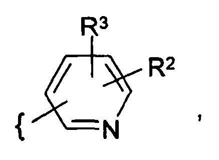
R¹ CN oder F,
R² H, Hai, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-,
Het unsubstituiertes oder einfach durch Hal oder COOA' substituiertes Chromen-2-on-yl, Thienyl, Pyridinyl, Pyrimidinyl, Indolyl, Furyl, Pyrrolyl, Isoxazolyl, Imidazolyl, Thiazolyl, Triazolyl, Tetrazolyl, Chinolyl oder Isochinolyl,
Ar unsubstituiertes oder ein-, zwei- oder dreifach durch Hal substituiertes Phenyl,
p 0 oder 1,
bedeuten,
sowie ihre pharmazeutisch verwendbaren Salze, Solvate und Stereoisomere, einschließlich deren Mischungen in allen Verhältnissen.

7. Verbindungen gemäß einem oder mehreren der Ansprüche 1-6, worin
X 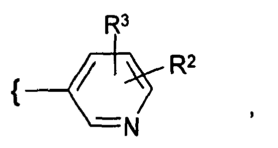
R¹ CN oder F,
R² H, Hal, A, SA, CN, CONH₂, COOA', -O-[C(R⁴R^{4'})]ₚAr, [C(R⁴R^{4'})]ₚAr oder [C(R⁴R^{4'})]ₚHet,
R³ H, A, Hal oder CN,
R² und R³ zusammen auch -CH=CH-CH=CH-,
Ar Phenyl,
p 0 oder 1,
bedeuten,
sowie ihre pharmazeutisch verwendbaren Salze, Solvate und Stereoisomere, einschließlich deren Mischungen in allen Verhältnissen.

8. Verbindungen gemäß einem oder mehreren der Ansprüche 1-7, worin
X 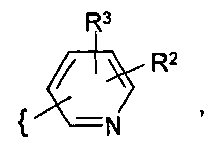
R¹ CN oder F,
R² H, Hal, A, SA, CN, CONH₂, COOA', -O-[C(R⁴R^{4'})]ₚAr, [C(R⁴R^{4'})]ₚAr,
R³ H, A, Hal oder CN,
R² und R³ zusammen auch -CH=CH-CH=CH-,
R⁴, R^{4'} H,
Ar Phenyl,
p 0 oder 1,
bedeuten,
sowie ihre pharmazeutisch verwendbaren Salze, Solvate und Stereoisomere, einschließlich deren Mischungen in allen Verhältnissen.

9. Verbindungen nach Anspruch 1
3-{4-[(5-Brom-pyridin-3-ylmethyl)-amino]-butyl}-1*H*-indol-5-carbonitril,
3-{4-[(Pyridin-3-ylmethyl)-amino]-butyl}-1*H*-indol-5-carbonitril,
3-{4-[(Pyridin-2-ylmethyl)-amino]-butyl}-1*H*-indol-5-carbonitril,
3-{4-[(Pyridin-4-ylmethyl)-amino]-butyl}-1*H*-indol-5-carbonitril,
3-{4-{[5-{4-Fluorphenyl)-pyridin-3-ylmethyl]-amino}-butyl)-1*H-*indol-5-carbonitril,
3-{4-[(Chinolin-3-ylmethyl)-amino]-butyl}-1*H*-indol-5-carbonitril,
3-[4-(2-Pyridin-4-yl-ethylamino]-butyl]-1*H*-indol-5-carbonitril,
3-{4-[(2,6-Dichlor-pyridin-4-ylmethyl)-amino]-butyl}-1*H*-indol-5-carbonitril,
3-{4-[(2-Chlor-6-methyl-pyridin-4-ylmethyl)-amino]-butyl}-1*H-*indol-5-carbonitril,
3-{4-[(2-Chlor-pyridin-4-ylmethyl)-amino]-butyl}-1*H*-indol-5-carbonitril,
3-{4-[(2-Methyl-pyridin-3-ylmethyl)-amino]-butyl}-1*H*-indol-5-carbonitril,
3-{4-[(6-Chlor-pyridin-3-ylmethyl)-amino]-butyl}-1*H*-indol-5-carbonitril,
3-(4-{[2-(4-Chlor-phenoxy)-pyridin-3-ylmethyl]-amino}-butyl)-1*H-*indol-5-carbonitril,
3-{4-[(2-Methylsulfanyl-pyridin-3-ylmethyl)-amino]-butyl}-1*H-*indol-5-carbonitril,
3-{4-[(2,5-Dichlor-pyridin-3-ylmethyl)-amino]-butyl}-1*H*-indol-5-carbonitril,
3-{4-[(2,6-Dichlor-pyridin-3-ylmethyl)-amino]-butyl}-1*H*-indol-5-carbonitril,
3-{4-[(5-Methyl-pyridin-3-ylmethyl)-amino]-butyl}-1*H*-indol-5-carbonitril,
3-{4-[(6-Trifluormethyl-pyridin-3-ylmethyl)-amino]-butyl}-1*H*-indol-5-carbonitril,
3-{4-[(Chinolin-2-ylmethyl)-amino]-butyl}-1*H*-indol-5-carbonitril,
3-{4-[(4-Phenyl-pyridin-2-ylmethyl)-amino]-butyl}-1*H*-indol-5-carbonitril,
3-{4-[(Chinolin-4-ylmethyl)-amino]-butyl}-1*H*-indol-5-carbonitril,
3-{4-[(4-Phenyl-pyridin-3-ylmethyl)-amino]-butyl}-1*H*-indol-5-carbonitril,
3-{4-[(5-Cyan-6-methylsulfanyl-pyridin-2-ylmethyl)-amino]-butyl}-1*H*-indol-5-carbonitril,
3-{4-[(5-Phenyl-pyridin-3-ylmethyl)-amino]-butyl}-1*H*-indol-5-carbonitril,
3-{4-[(5-Phenyl-pyridin-2-ylmethyl)-amino]-butyl}-1*H*-indol-5-carbonitril,
3-[4-(Methyl-pyridin-3-ylmethyl-amino)-butyl]-1*H*-indol-5-carbonitril,
sowie ihre pharmazeutisch verwendbaren Salze, Solvate und Stereoisomere, einschließlich deren Mischungen in allen Verhältnissen.

10. Verfahren zur Herstellung von Verbindungen der Formel I nach den Ansprüchen 1-9 sowie ihrer pharmazeutisch verwendbaren Salze, Solvate und Stereoisomere, **dadurch gekennzeichnet, daß** man
a) eine Verbindung der Formel II 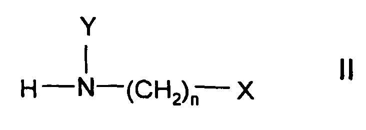 worin
X, Y und n die in Anspruch 1 angegebenen Bedeutungen haben, mit einer Verbindung der Formel III 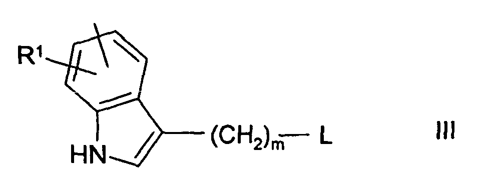 worin
L Cl, Br oder I bedeutet und R¹, R^{1'} und m die in Anspruch 1 angegebenen Bedeutungen haben,
umsetzt,
oder
b) eine Verbindung der Formel IV
X-(CH₂)ₙ-L IV
worin
L Cl, Br oder I bedeutet und X und n die in Anspruch 1 angegebenen Bedeutungen haben,
mit einer Verbindung der Formel V 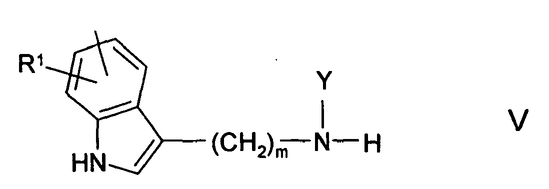 worin
R¹, Y und m die in Anspruch 1 angegebenen Bedeutungen haben,
umsetzt,
und/oder
eine basische oder saure Verbindung der Formel I durch Behandeln mit einer Säure oder Base in eines ihrer Salze und/oder Solvate umwandelt.

11. Verbindungen der Formel I nach einem oder mehreren der Ansprüche 1 bis 9 und ihre physiologisch unbedenklichen Salze oder Solvate als 5HT_{1A}- und/oder 5HT_{1D}- Agonisten und als Inhibitoren der 5-HT-Wiederaufnahme.

12. Arzneimittel, enthaltend mindestens eine Verbindung der Formel I nach einem oder mehreren der Ansprüche 1 bis 9 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

13. Arzneimittel enthaltend mindestens eine Verbindung der Formel I gemäß einem oder mehreren der Ansprüche 1 bis 9 und/oder ihre pharmazeutisch verwendbaren Salze, Solvate und Stereoisomere, einschließlich deren Mischungen in allen Verhältnissen, und mindestens einen weiteren Arzneimittelwirkstoff.

14. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels.

15. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels zur Bekämpfung von Krankheiten, die mit dem Serotonin-Neurotransmittersystem verbunden sind und bei denen hochaffine Serotoninrezeptoren (5-HT_{1A}-Rezeptoren) und/oder 5-HT_{1D} -Rezeptoren beteiligt sind.

16. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels als Anxiolytikum, Antidepressivum, Neuroleptikum und/oder Antihypertensivum.

17. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels zur Bekämpfung von Psychosen, der Symptome von Morbus-Alzheimer, krankhaften Angstzuständen, Übererregung, Hyperaktivität, Ausmerksamkeitsstörungen bei Kindern und Jugendlichen, tiefgreifende Entwicklungstörungen und Störungen des Sozialverhaltens mit geistiger Retardierung, Depression, Zwangserkrankungen im engeren (OCD) und weiteren Sinne (OCSD), sexuelle Funktionsstörungen, Schlafstörungen, Störungen in der Nahrungsaufnahme, sowie solcher Psychiatrischer Symptome in Rahmen der Altersdemenz und der Demenz vom Alzheimer-Typ, zur Verminderung von kognitiven Leistungsstörungen oder zur Prophylaxe und Kontrolle von Hirninfarkten (Apoplexia cerebri), zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden.

18. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels zur Bekämpfung von Schizophrenie.

19. Verwendung von Verbindungen der Formel I nach einem oder mehreren der Ansprüche 1 bis 9 und/oder ihrer pharmazeutisch verwendbaren Salze, Solvate und Stereoisomere, einschließlich deren Mischungen in allen Verhältnissen, zur Herstellung eines Arzneimittels zur Bekämpfung von neurodegenerativen Erkrankungen.

20. Verwendung nach Anspruch 19, wobei es sich bei den Erkrankungen um Lathyrismus, Alzheimer, Parkinson, und Huntington handelt.

21. Set (Kit), bestehend aus getrennten Packungen von
(a) einer wirksamen Menge an einer Verbindung der Formel 1 gemäß einem oder mehreren der Ansprüche 1 bis 9 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

## Claims

1. Compounds of the formula I 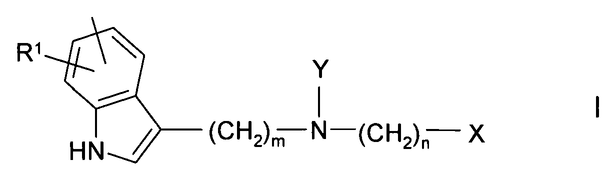 in which
X denotes 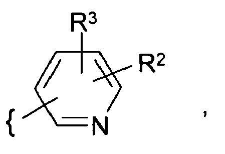
Y denotes H or A',
R¹ denotes CN or F,
R² denotes H, A, Hal, OH, OA, SA, COOH, COOA', CHO, COA', SO₂A', NH₂, NHA, NA₂, CH₂NA₂, NHCOA, NHCOAr, NHCOOA, NHSO₂A, NHSO₂Ar, CH₂NHSO₂A, NHCONH₂, NHCONHA, NHCONA₂, NHCONHAr, CONH₂, CONHA, CONA₂, SO₂NH₂, SO₂NHA, SO₂NA₂, CH₂SO₂NH₂, CH₂SO₂NHA, CH₂SO₂NA₂, [C(R⁴R^{4'})]ₚCN, [C(R⁴R^{4'})]ₚCF₃, [C(R⁴R^{4'})ₚCOR⁴, [C(R⁴R^{4'})]ₚAr, -O-[C(R⁴R⁴)]ₚAr or [C(R⁴R^{4'})]ₚHet,
R³ denotes H, A, [C(R⁴R^{4'})]ₚAr or [C(R⁴R^{4'})]ₚHet,
R² and R³ together also denote -CH=CH-CH=CH-, -CH=CH-CH₂₋CH₂- or -CH₂-CH₂-CH=CH-, in which 1 or 2 CH and/or CH₂ units may be replaced by N and/or 1, 2, 3 or 4 H atoms may be substituted by Hal, A, OH, OA, NH₂, NO₂, CN, COOH, COOA, CONH₂, NHCOA, NHCONH₂, NHSO₂A, CHO, COA, SO₂NH₂ or S(O)ₒA, -O-CH₂-O- or -O-CH₂-CH₂-O-,
R⁴, R^{4'} each, independently of one another, denote H, A, OH, OA, NH₂, NHA, NA₂ or NHCOOA',
Ar denotes phenyl, naphthyl or biphenyl, each of which is unsubstituted or mono-, di- or trisubstituted by Hal, A, OH, OA, NH₂, NO₂, CN, COOH, COOA, CONH₂, NHCOA, NHCONH₂, NHSO₂A, CHO, COA, SO₂NH₂ or S(O)ₒA,
Het denotes a mono- or bicyclic saturated, unsaturated or aromatic heterocycle having 1 to 4 N, O and/or S atoms, which may be unsubstituted or mono-, di- or trisubstituted by carbonyl oxygen, =S, =NH, Hal, A, -(CH₂)ₒ-Ar, -(CH₂)ₒ-cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ and/or S(O)ₒA,
A denotes unbranched or branched alkyl having 1-10 C atoms, in which one or two CH₂ groups may be replaced by -CH=CH- groups and/or 1-7 H atoms may also be replaced by F and/or Cl,
A' denotes alkyl having 1 to 6 C atoms or benzyl,
Hal denotes F, Cl, Br or I and
m denotes 2, 3, 4, 5 or 6,
n denotes 1, 2, 3 or 4,
o denotes 0, 1 or 2,
p denotes 0, 1, 2, 3, 4, 5 or 6,
and pharmaceutically usable salts, solvates and stereoisomers thereof, including mixtures thereof in all ratios.

2. Compounds according to Claim 1 in which
R⁴, R^{4'} denote H,
and pharmaceutically usable salts, solvates and stereoisomers thereof, including mixtures thereof in all ratios.

3. Compounds according to Claim 1 or 2 in which
R² denotes H, Hal, A, SA, CN, CONH₂, COOA', -O-[C(R⁴R^{4'})]ₚAr, [C(R⁴R^{4'})]ₚAr or [C(R⁴R^{4'})]pHet,
R³ denotes H, A, Hal or CN,
R² and R³ together also denote -CH=CH-CH=CH-,
and pharmaceutically usable salts, solvates and stereoisomers thereof, including mixtures thereof in all ratios.

4. Compounds according to one or more of Claims 1-3 in which
Het denotes chromen-2-onyl, thienyl, pyridinyl, pyrimidinyl, indolyl, furyl, pyrrolyl, isoxazolyl, imidazolyl, thiazolyl, triazolyl, tetrazolyl, quinolyl or isoquinolyl, each of which is unsubstituted or monosubstituted by Hal or COOA',
and pharmaceutically usable salts, solvates and stereoisomers thereof, including mixtures thereof in all ratios.

5. Compounds according to one or more of Claims 1-4 in which
Ar denotes phenyl which is unsubstituted or mono-, di- or trisubstituted by Hal,
and pharmaceutically usable salts, solvates and stereoisomers thereof, including mixtures thereof in all ratios.

6. Compounds according to one or more of Claims 1-5 in which
X denotes 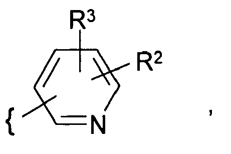
R¹ denotes CN or F,
R² denotes H, Hal, A, SA, CN, CONH₂, COOA', -O-[C(R⁴R^{4'})]ₚAr, [C(R⁴R^{4'})]ₚAr or [C(R⁴R^{4'})]ₚHet,
R³ denotes H, A, Hal or CN,
R² and R³ together also denote -CH=CH-CH=CH-,
Het denotes chromen-2-onyl, thienyl, pyridinyl, pyrimidinyl, indolyl, furyl, pyrrolyl, isoxazolyl, imidazolyl, thiazolyl, triazolyl, tetrazolyl, quinolyl or isoquinolyl, each of which is unsubstituted or monosubstituted by Hal or COOA',
Ar denotes phenyl which is unsubstituted or mono-, di- or trisubstituted by Hal,
p denotes 0 or 1,
and pharmaceutically usable salts, solvates and stereoisomers thereof, including mixtures thereof in all ratios.

7. Compounds according to one or more of Claims 1-6 in which
X denotes 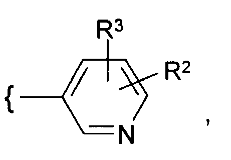
R¹ denotes CN or F,
R² denotes H, Hal, A, SA, CN, CONH₂, COOA', -O-[C(R⁴R^{4'})]ₚAr, [C(R⁴R^{4'})]ₚAr or [C(R⁴R^{4'})]ₚHet,
R³ denotes H, A, Hal or CN,
R² and R³ together also denote -CH=CH-CH=CH-,
Ar denotes phenyl,
p denotes 0 or 1,
and pharmaceutically usable salts, solvates and stereoisomers thereof, including mixtures thereof in all ratios.

8. Compounds according to one or more of Claims 1-7 in which
X denotes 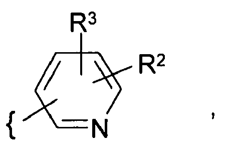
R¹ denotes CN or F,
R² denotes H, Hal, A, SA, CN, CONH₂, COOA', -O-[C(R⁴R^{4'})]ₚAr, [C(R⁴R^{4'})]ₚAr,
R³ denotes H, A, Hal or CN,
R² and R³ together also denote -CH=CH-CH=CH-,
R⁴, R^{4'} denote H,
Ar denotes phenyl,
p denotes 0 or 1,
and pharmaceutically usable salts, solvates and stereoisomers thereof, including mixtures thereof in all ratios.

9. Compounds according to Claim 1
3-{4-[(5-bromopyridin-3-ylmethyl)amino]butyl}-1*H*-indole-5-carbonitrile,
3-{4-[(pyridin-3-ylmethyl)amino]butyl}-1*H*-indole-5-carbonitrile,
3-{4-[(pyridin-2-ylmethyl)amino]butyl}-1*H*-indole-5-carbonitrile,
3-{4-[(pyridin-4-ylmethyl)amino]butyl}-1*H*-indole-5-carbonitrile,
3-(4-{[5-(4-fluorophenyl)pyridin-3-ylmethyl]amino}butyl)-1*H-*indole-5-carbonitrile,
3-{4-[(quinolin-3-ylmethyl)amino]butyl}-1*H*-indole-5-carbonitrile,
3-[4-(2-pyridin-4-ylethylamino]butyl)-1*H*-indole-5-carbonitrile,
3-{4-[(2,6-dichloropyridin-4-ylmethyl)amino]butyl}-1*H*-indole-5-carbonitrile,
3-{4-[(2-chloro-6-methylpyridin-4-ylmethyl)amino]butyl}-1*H-*indole-5-carbonitrile,
3-{4-[(2-chloropyridin-4-ylmethyl)amino]butyl}-1*H*-indole-5-carbonitrile,
3-{4-[(2-methylpyridin-3-ylmethyl)amino]butyl}-1*H*-indole-5-carbonitrile,
3-{4-[(6-chloropyridin-3-ylmethyl)amino]butyl}-1*H*-indole-5-carbonitrile,
3-(4-{[2-(4-chlorophenoxy)pyridin-3-ylmethyl]amino}butyl)-1*H-*indole-5-carbonitrile,
3-{4-[(2-methylsulfanylpyridin-3-ylmethyl)amino]butyl}-1*H*-indole-5-carbonitrile,
3-{4-[(2,5-dichloropyridin-3-ylmethyl)amino]butyl}-1*H*-indole-5-carbonitrile,
3-{4-[(2,6-dichloropyridin-3-ylmethyl)amino]butyl}-1*H*-indole-5-carbonitrile,
3-{4-[(5-methylpyridin-3-ylmethyl)amino]butyl}-1*H*-indole-5-carbonitrile,
3-{4-[(6-trifluoromethylpyridin-3-ylmethyl)amino]butyl}-1*H*-indole-5-carbonitrile,
3-{4-[(quinolin-2-ylmethyl)amino]butyl}-1*H*-indole-5-carbonitrile,
3-{4-[(4-phenylpyridin-2-ylmethyl)amino]butyl}-1*H*-indole-5-carbonitrile,
3-{4-[(quinolin-4-ylmethyl)amino]butyl}-1*H*-indole-5-carbonitrile,
3-{4-[(4-phenylpyridin-3-ylmethyl)amino]butyl}-1*H*-indole-5-carbonitrile,
3-{4-[(5-cyano-6-methylsulfanylpyridin-2-ylmethyl)amino]butyl}-1*H*-indole-5-carbonitrile,
3-{4-[(5-phenylpyridin-3-ylmethyl)amino]butyl}-1*H*-indole-5-carbonitrile,
3-{4-[(5-phenylpyridin-2-ylmethyl)amino]butyl}-1*H*-indole-5-carbonitrile,
3-[4-(methylpyridin-3-ylmethylamino)butyl]-1*H*-indole-5-carbonitrile,
and pharmaceutically usable salts, solvates and stereoisomers thereof, including mixtures thereof in all ratios.

10. Process for the preparation of compounds of the formula I according to Claims 1-9 and pharmaceutically usable salts, solvates and stereoisomers thereof, **characterised in that**
a) a compound of the formula II 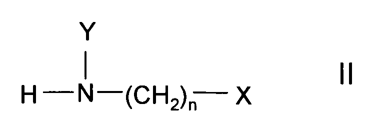 in which
X, Y and n have the meanings indicated in Claim 1, is reacted with a compound of the formula III 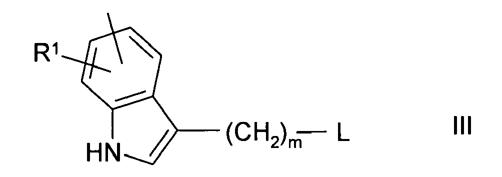 in which
L denotes Cl, Br or I and R¹, R^{1'} and m have the meanings indicated in Claim 1,
or
b) a compound of the formula IV
X-(CH₂)ₙ-L IV
in which
L denotes Cl, Br or I and X and n have the meanings indicated in Claim 1,
is reacted with a compound of the formula V 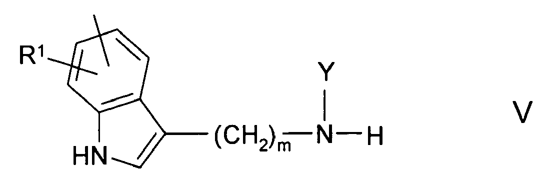 in which
R¹, Y and m have the meanings indicated in Claim 1,
and/or
a basic or acidic compound of the formula I is converted into one of its salts and/or solvates by treatment with an acid or base.

11. Compounds of the formula I according to one or more of Claims 1 to 9 and physiologically acceptable salts or solvates thereof as 5HT_{1A} and/or 5HT_{1D} agonists and as inhibitors of 5-HT reuptake.

12. Medicaments comprising at least one compound of the formula I according to one or more of Claims 1 to 9 and/or pharmaceutically usable salts, solvates and stereoisomers thereof, including mixtures thereof in all ratios, and optionally excipients and/or adjuvants.

13. Medicaments comprising at least one compound of the formula I according to one or more of Claims 1 to 9 and/or pharmaceutically usable salts, solvates and stereoisomers thereof, including mixtures thereof in all ratios, and at least one further medicament active ingredient.

14. Use of compounds of the formula I according to one or more of Claims 1 to 9 and/or pharmaceutically usable salts, solvates and stereoisomers thereof, including mixtures thereof in all ratios, for the preparation of a medicament.

15. Use of compounds of the formula I according to one or more of Claims 1 to 9 and/or pharmaceutically usable salts, solvates and stereoisomers thereof, including mixtures thereof in all ratios, for the preparation of a medicament for combating diseases which are associated with the serotonin neurotransmitter system and in which high-affinity serotonin receptors (5-HT_{1A} receptors) and/or 5-HT_{1D} receptors are involved.

16. Use of compounds of the formula I according to one or more of Claims 1 to 9 and/or pharmaceutically usable salts, solvates and stereoisomers thereof, including mixtures thereof in all ratios, for the preparation of a medicament as anxiolytic, antidepressant, neuroleptic and/or antihypertensive.

17. Use of compounds of the formula I according to one or more of Claims 1 to 9 and/or pharmaceutically usable salts, solvates and stereoisomers thereof, including mixtures thereof in all ratios, for the preparation of a medicament for combating psychoses, the symptoms of Alzheimer's disease, pathological anxiety states, overexcitation, hyperactivity, attention disorders in children and youths, severe developmental disorders and disorders of social behaviour with mental retardation, depression, obsessive-compulsive disorders in the narrower (OCD) and broader sense (OCSD), sexual dysfunctions, sleeping disorders, disorders in nutrient uptake, and psychiatric symptoms as part of age dementia and dementia of the Alzheimer's type, for reducing defects in cognitive ability, or for the prophylaxis and control of cerebral infarctions (apoplexia cerebri), for the treatment of extrapyramidal motor diseases, for the treatment of side effects which occur in the treatment of extrapyramidal motor diseases with conventional anti-Parkinson's medicaments, or for the treatment of extrapyramidal symptoms (EPS) induced by neuroleptics.

18. Use of compounds of the formula I according to one or more of Claims 1 to 9 and/or pharmaceutically usable salts, solvates and stereoisomers thereof, including mixtures thereof in all ratios, for the preparation of a medicament for combating schizophrenia.

19. Use of compounds of the formula I according to one or more of Claims 1 to 9 and/or pharmaceutically usable salts, solvates and stereoisomers thereof, including mixtures thereof in all ratios, for the preparation of a medicament for combating neurodegenerative diseases.

20. Use according to Claim 19, where the diseases are lathyrism, Alzheimer's, Parkinson's and Huntington's.

21. Set (kit) consisting of separate packs of
(a) an effective amount of a compound of the formula I according to one or more of Claims 1 to 9 and/or pharmaceutically usable salts, solvates and stereoisomers thereof, including mixtures thereof in all ratios,
and
(b) an effective amount of a further medicament active ingredient.

## Revendications

1. Composés de la formule I 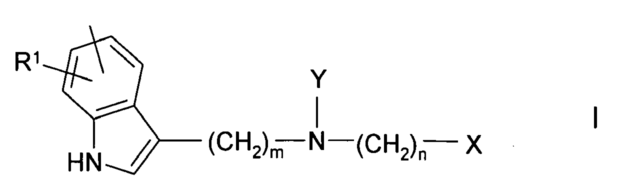 dans laquelle
X représente 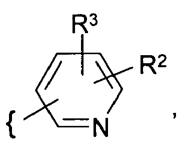
Y représente H ou A',
R¹ représente CN ou F,
R² représente H, A, Hal, OH, OA, SA, COOH, COOA', CHO, COA', SO₂A', NH₂, NHA, NA₂, CH₂NA₂, NHCOA, NHCOAr, NHCOOA, NHSO₂A, NHSO₂Ar, CH₂NHSO₂A, NHCONH₂, NHCONHA, NHCONA₂, NHCONHAr, CONH₂, CONHA, CONA₂, SO₂NH₂, SO₂NHA, SO₂NA₂, CH₂SO₂NH₂, CH₂SO₂NHA, CH₂SO₂NA₂, [C(R⁴R^{4'})]ₚCN, [C(R⁴R^{4'})]ₚCF₃, [C(R⁴R^{4'})]ₚCOR⁴, [C(R⁴R^{4'})]ₚAr, -O-[C(R⁴R^{4'})]ₚAr ou [C(R⁴R^{4'})]ₚHet,
R³ représente H, A, [C(R⁴R^{4'})]ₚAr ou [C(R⁴R^{4'})]ₚHet,
R² et R³ représentent également ensemble -CH=CH-CH=CH-, -CH=CH-CH₂-CH₂- ou -CH₂-CH₂-CH=CH-, où 1 ou 2 unités CH et/ou CH₂ peuvent être remplacées par N et/ou 1, 2, 3 ou 4 atomes de H peuvent être substitués par Hal, A, OH, OA, NH₂, NO₂, CN, COOH, COOA, CONH₂, NHCOA, NHCONH₂, NHSO₂A, CHO, COA, SO₂NH₂ ou S(O)ₒA,
-O-CH₂-O- ou -O-CH₂-CH₂-O-,
R⁴, R^{4'} chacun indépendamment de l'autre, représentent H, A, OH, OA, NH₂, NHA, NA₂ ou NHCOOA',
Ar représente phényle, naphthyle ou biphényle, dont chacun est non substitué ou mono-, di- ou trisubstitué par Hal, A, OH, OA, NH₂, NO₂, CN, COOH, COOA, CONH₂, NHCOA, NHCONH₂, NHSO₂A, CHO, COA, SO₂NH₂ ou S(O)ₒA,
Het représente un hétérocycle saturé, non saturé ou aromatique mono- ou bicyclique comportant de 1 à 4 atomes de N, O et/ou S, qui peut être non substitué ou mono-, di- ou trisubstitué par oxygène carbonyle, =S, =NH, Hal, A, -(CH₂)ₒ-Ar, -(CH₂)ₒ-cycloalkyle, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ₋NHCOA, NHCONH₂, -(CH₂)ₒ-NHSO₂A, CHO, COA', SO₂NH₂ et/ou S(O)ₒA,
A représente un alkyle non ramifié ou ramifié comportant de 1 à 10 atomes de C, où un ou deux groupes CH₂ peuvent être remplacés par des groupes -CH=CH- et/ou 1 à 7 atomes de H peuvent également être remplacés par F et/ou CI,
A' représente alkyle comportant de 1 à 6 atomes de C ou benzyle,
Hal représente F, Cl, Br ou I et
m représente 2, 3, 4, 5 ou 6,
n représente 1, 2, 3 ou 4,
o représente 0, 1 ou 2,
p représente 0, 1, 2, 3, 4, 5 ou 6,
et leurs sels, solvates et stéréo-isomères pharmaceutiquement utilisables, y compris des mélanges afférents selon tous rapports.

2. Composés selon la revendication 1 selon lesquels
R⁴, R^{4'} représentent H,
et leurs sels, solvates et stéréo-isomères pharmaceutiquement utilisables, y compris des mélanges afférents selon tous rapports.

3. Composés selon la revendication 1 ou 2 selon lesquels
R² représente H, Hal, A, SA, CN, CONH₂, COOA', -O-[C(R⁴R^{4'})]ₚAr, [C(R⁴R^{4'})]ₚAr ou [C(R⁴R^{4'})]ₚHet,
R³ représente H, A, Hal ou CN,
R² et R³ représentent également ensemble -CH=CH-CH=CH-,
et leurs sels, solvates et stéréo-isomères pharmaceutiquement utilisables, y compris des mélanges afférents selon tous rapports.

4. Composés selon une ou plusieurs des revendications 1 à 3 selon lesquels
Het représente chromen-2-onyle, thiényle, pyridinyle, pyrimidinyle, indolyle, furyle, pyrrolyle, isoxazolyle, imidazolyle, thiazolyle, triazolyle, tétrazolyle, quinolyle ou isoquinolyle, dont chacun est non substitué ou monosubstitué par Hal ou COOA',
et leurs sels, solvates et stéréo-isomères pharmaceutiquement utilisables, y compris des mélanges afférents selon tous rapports.

5. Composés selon une ou plusieurs des revendications 1 à 4 selon lesquels
Ar représente phényle qui est non substitué ou mono-, di- ou tri-substitué par Hal,
et leurs sels, solvates et stéréo-isomères pharmaceutiquement utilisables, y compris des mélanges afférents selon tous rapports.

6. Composés selon une ou plusieurs des revendications 1 à 5 selon lesquels
X représente 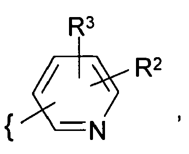
R¹ représente CN ou F,
R² représente H, Hal, A, SA, CN, CONH₂, COOA', -O-[C(R⁴R^{4'})]ₚAr, [C(R⁴R^{4'})]ₚAr ou [C(R⁴R^{4'})]ₚHet,
R³ représente H, A, Hal ou CN,
R² et R³ représentent également ensemble -CH=CH-CH=CH-,
Het représente chromen-2-onyle, thiényle, pyridinyle, pyrimidinyle, indolyle, furyle, pyrrolyle, isoxazolyle, imidazolyle, thiazolyle, triazolyle, tétrazolyle, quinolyle ou isoquinolyle, dont chacun est non substitué ou monosubstitué par Hal ou COOA',
Ar représente phényle qui est non substitué ou mono-, di- ou tri-substitué par Hal,
p représente 0 ou 1,
et leurs sels, solvates et stéréo-isomères pharmaceutiquement utilisables, y compris des mélanges afférents selon tous rapports.

7. Composés selon une ou plusieurs des revendications 1 à 6 selon lesquels
X représente 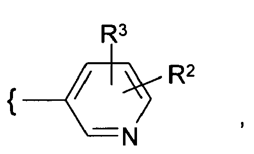
R¹ représente CN ou F,
R² représente H, Hal, A, SA, CN, CONH₂, COOA', -O-[C(R⁴R^{4'})]ₚAr, [C(R⁴R^{4'})]ₚAr ou [C(R⁴R^{4'})]ₚHet,
R³ représente H, A, Hal ou CN,
R² et R³ représentent également ensemble -CH=CH-CH=CH-,
Ar représente phényle,
p représente 0 ou 1,
et leurs sels, solvates et stéréo-isomères pharmaceutiquement utilisables, y compris des mélanges afférents selon tous rapports.

8. Composés selon une ou plusieurs des revendications 1 à 7 selon lesquels
X représente 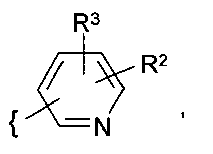
R¹ représente CN ou F,
R² représente H, Hal, A, SA, CN, CONH₂, COOA', -O-[C(R⁴R^{4'})]ₚAr, [C(R⁴R^{4'})]ₚAr,
R³ représente H, A, Hal ou CN,
R² et R³ représentent également ensemble -CH=CH-CH=CH-,
R⁴, R^{4'} représentent H,
Ar représente phényle,
p représente 0 ou 1,
et leurs sels, solvates et stéréo-isomères pharmaceutiquement utilisables, y compris des mélanges afférents selon tous rapports.

9. Composés selon la revendication 1
3-{4-[(5-bromopyridine-3-ylméthyl)amino]butyl}-1*H*-indole-5-carbonitrile,
3-{4-[(pyridine-3-ylméthyl)amino]butyl}-1*H*-indole-5-carbonitrile,
3-{4-[(pyridine-2-ylméthyl)amino]butyl}-1*H*-indole-5-carbonitrile,
3-{4-[(pyridine-4-ylméthyl)amino]butyl}-1*H*-indole-5-carbonitrile,
3-(4-{[5-(4-fluorophényl)pyridine-3-ylméthyl]amino}butyl)-1*H-*indole-5-carbonitrile,
3-{4-[(quinoline-3-ylméthyl)amino]butyl}-1*H*-indole-5-carbonitrile,
3-[4-(2-pyridine-4-yléthylamino]butyl)-1*H*-indole-5-carbonitrile,
3-{4-[(2,6-dichloropyridine-4-ylméthyl)amino]butyl}-1*H*-indole-5-carbonitrile,
3-{4-[(2-chloro-6-méthylpyridine-4-ylméthyl)amino]butyl}-1*H-*indole-5-carbonitrile,
3-{4-[(2-chloropyridine-4-ylméthyl)amino]butyl}-1*H*-indole-5-carbonitrile,
3-{4-[(2-méthylpyridine-3-ylméthyl)amino]butyl}-1*H*-indole-5-carbonitrile,
3-{4-[(6-chloropyridine-3-ylméthyl)amino]butyl}-1*H*-indole-5-carbonitrile,
3-(4-{[2-(4-chlorophénoxy)pyridine-3-ylméthyl]amino}butyl)-1*H-*indole-5-carbonitrile,
3-{4-[(2-méthylsulfanylpyridine-3-ylméthyl)amino]butyl}-1*H-*indole-5-carbonitrile,
3-{4-[(2,5-dichloropyridine-3-ylméthyl)amino]butyl}-1*H*-indole-5-carbonitrile,
3-{4-[(2,6-dichloropyridine-3-ylméthyl)amino]butyl}-1*H*-indole-5-carbonitrile,
3-{4-[(5-méthylpyridine-3-ylméthyl)amino]butyl}-1*H*-indole-5-carbonitrile,
3-{4-[(6-trifluorométhylpyridine-3-ylméthyl)amino]butyl}-1*H-*indole-5-carbonitrile,
3-{4-[(quinoline-2-ylméthyl)amino]butyl}-1*H*-indole-5-carbonitrile,
3-{4-[(4-phénylpyridine-2-ylméthyl)amino]butyl}-1*H*-indole-5-carbonitrile,
3-{4-[(quinoline-4-ylméthyl)amino]butyl}-1*H*-indole-5-carbonitrile,
3-{4-[(4-phénylpyridine-3-ylméthyl)amino]butyl}-1*H*-indole-5-carbonitrile,
3-{4-[(5-cyano-6-méthylsulfanylpyridine-2-ylméthyl)amino]butyl}-1*H*-indole-5-carbonitrile,
3-(4-[(5-phénylpyridine-3-ylméthyl)amino]butyl}-1*H*-indole-5-carbonitrile,
3-{4-[(5-phénylpyridine-2-ylméthyl)amino]butyl}-1*H*-indole-5-carbonitrile,
3-[4-(méthylpyridine-3-ylméthylamino)butyl]-1*H*-indole-5-carbonitrile,
et leurs sels, solvates et stéréo-isomères pharmaceutiquement utilisables, y compris des mélanges afférents selon tous rapports.

10. Procédé pour la préparation de composés de la formule I selon les revendications 1 à 9 et de leurs sels, solvates et stéréo-isomères pharmaceutiquement utilisables, **caractérisé en ce que**
a) un composé de la formule II 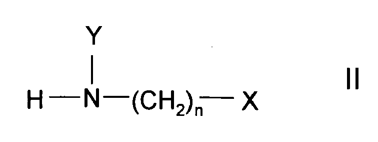 dans laquelle
X, Y et n présentent les significations indiquées selon la revendication 1,
est amené à réagir avec un composé de la formule III 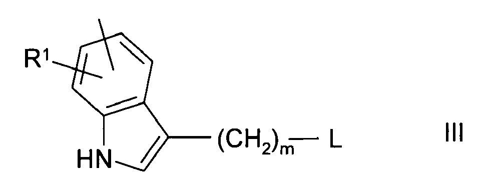 dans laquelle
L représente Cl, Br ou 1 et R¹, R^{1'} et m présentent les significations indiquées selon la revendication 1,
ou
b) un composé de la formule IV
X-(CH₂)ₙ-L IV
dans laquelle
L représente Cl, Br ou I et X et n présentent les significations indiquées selon la revendication 1,
est amené à réagir avec un composé de la formule V 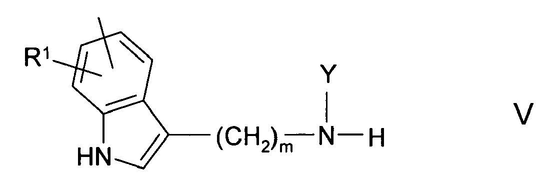 dans laquelle
R¹, Y et m présentent les significations indiquées selon la revendication 1,
et/ou
un composé basique ou acide de la formule I est converti selon l'un de ses sels et/ou solvates par traitement avec un acide ou une base.

11. Composés de la formule I selon une ou plusieurs des revendications 1 à 9 et leurs sels ou solvates physiologiquement acceptables en tant qu'agonistes 5HT_{1A} et/ou 5HT_{1D} et en tant qu'inhibiteurs de réadmission de 5-HT.

12. Médicaments comprenant au moins un composé de la formule I selon une ou plusieurs des revendications 1 à 9 et/ou leurs sels, solvates et stéréo-isomères pharmaceutiquement utilisables, y compris des mélanges afférents selon tous rapports, et en option, des excipients et/ou adjuvants.

13. Médicaments comprenant au moins un composé de la formule I selon une ou plusieurs des revendications 1 à 9 et/ou leurs sels, solvates et stéréo-isomères pharmaceutiquement utilisables, y compris des mélanges afférents selon tous rapports, et au moins un autre ingrédient actif de médicament.

14. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament.

15. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament pour combattre des maladies qui sont associées au système neurotransmetteur de sérotonine et selon laquelle des récepteurs de sérotonine haute affinité (récepteurs 5-HT_{1A}) et/ou des récepteurs 5-HT_{1D} sont mis en jeu.

16. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament en tant qu'anxiolytique, antidépresseur, neuroleptique et/ou antihypertenseur.

17. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament pour combattre les psychoses, les symptômes de la maladie d'Alzheimer, les états d'anxiété pathologiques, la surexcitation, l'hyperactivité, les troubles de l'attention chez les enfants et les adolescents, les troubles sévères du développement et les troubles du comportement social avec retard mental, la dépression, les troubles obsessionnels compulsifs au sens plus étroit (OCD) et au sens plus large (OCSD), les dysfonctionnements sexuels, les troubles du sommeil, les troubles de la prise alimentaire et les symptômes psychiatriques en tant que partie de la démence sénile et de la démence du type Alzheimer, pour réduire des défauts de la capacité cognitive, ou pour la prophylaxie et le contrôle des infarctus cérébraux (apoplexie cérébrale), pour le traitement des troubles moteurs extrapyramidaux, pour le traitement des effets secondaires qui surviennent au niveau du traitement des troubles moteurs extrapyramidaux avec des médicaments anti-Parkinsoniens classiques, ou pour le traitement de symptômes extrapyramidaux (EPS) induits par des neuroleptiques.

18. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament pour combattre la schizophrénie.

19. Utilisation de composés de la formule I selon une ou plusieurs des revendications 1 à 9 et/ou de leurs sels, solvates et stéréo-isomères pharmaceutiquement utilisables, y compris des mélanges afférents selon tous rapports, pour la préparation d'un médicament pour combattre les maladies neurodégénératives.

20. Utilisation selon la revendication 19, où les maladies sont lathyrisme, Alzheimer, Parkinson et Huntington.

21. Ensemble (kit) constitué par des packs séparés de
(a) une quantité efficace d'un composé de la formule I selon une ou plusieurs des revendications 1 à 9 et/ou de leurs sels, solvates et stéréo-isomères pharmaceutiquement utilisables, y compris des mélanges afférents selon tous rapports, et
(b) une quantité efficace d'un autre ingrédient actif de médicament.
